# EUROPEAN PATENT APPLICATION

(11) **EP 4 209 782 A1**
(43) Date of publication of application: **12.07.2023**
(21) Application number: 20952461.0
(22) Date of filing: 04.09.2020
(51) Int. Cl.: G01N 27/62, G01N 30/88

(54) **ANALYSIS METHOD AND ANALYSIS SYSTEM FOR BILE ACIDS, STEROLS, AND HORMONES**

(71) Applicant: Aderans Company Limited, Tokyo 160-8429 (JP); Kazusa DNA Research Institute, Kisarazu-shi, Chiba 292-0818 (JP)
(72) Inventor: IKEDA, Kazutaka, Kisarazu-shi, Chiba 292-0818 (JP); ISHIKAWA, Masaki, Kisarazu-shi, Chiba 292-0818 (JP); IGARASHI, Kohei, Tokyo 160-8429 (JP); OHTA, Atsuko, Tokyo 160-8429 (JP)
(74) Representative: Plasseraud IP
(86) International application number: PCT/JP2020/033613
(87) International publication number: WO 2022/049726

(57) **Abstract**

A technique for comprehensively analyzing bile acids, sterols, and hormones is achieved. A method for analyzing bile acids, sterols, and hormones includes: a step of separating a plurality of molecules selected from among bile acids, sterols, and hormones in a sample by reversed-phase liquid chromatography; and a step of ionizing the molecules that have been separated; and a step of detecting, through MS analysis, the molecules that have been ionized.

## Description

### Technical Field

The present invention relates to a method for analyzing bile acids, sterols, and hormones and a system for analyzing bile acids, sterols, and hormones.

### Background Art

Known are techniques in which specific components contained in, for example, blood and tissues collected from living bodies are detected as indicators (biomarkers) of health and diseases with use of a mass spectrometer (MS) to thereby predict or diagnose health conditions and diseases. Among such biological components, many bile acids, sterols, and hormones having cholesterol as a precursor have physiological activities, and are known to be associated with various diseases. Therefore, enabling comprehensive analysis of bile acids, sterols, and hormones is extremely important for efficient search and discovery of highly accurate biomarkers.

For example, Non-patent Literature 1 describes, as a method for analyzing components having cholesterol as a precursor, a method for carrying out MS analysis of cholesterol analogs, oxysterols, and vitamin Ds from human blood (serum). In addition, Non-patent Literature 2 describes a method for carrying out MS analysis of oxysterols using a derivatization method, Non-patent Literature 3 describes a method for carrying out MS analysis of steroids using a derivatization method, and Patent Literature 1 describes a method for carrying out MS analysis of steroid analogs and vitamin D3 analogs using a derivatization method.

### Citation List

### [Patent Literature]

[Patent Literature 1]
Japanese Patent Application Publication Tokukai No. 2007-132741

### [Non-patent Literature]

[Non-patent Literature 1]
   Jeffrey G. McDonald, Daniel D. Smith, Ashlee R. Stiles, David W. Russell, A comprehensive method for extraction and quantitative analysis of sterols and secosteroids from human plasma, Journal of Lipid Research 53(7):1399-409. 2012
[Non-patent Literature 2]
   Akira Honda, Kouwa Yamashita, Takashi Hara, Tadashi Ikegami, Teruo Miyazaki, Mutsumi Shirai, Guorong Xu, Mitsuteru Numazawa, Yasushi Matsuzaki, Highly sensitive quantification of key regulatory oxysterols in biological samples by LC-ESI-MS/MS, Journal of Lipid Research 50(2):350-7. 2009
[Non-patent Literature 3]
   Teng-Fei Yuan, Juan Le, Shao-Ting Wang, Yan Li, An LC/MS/MS method for analyzing the steroid metabolome with high accuracy and from small serum samples, Journal of Lipid Research 61(4):580-586. 2020

### Summary of Invention

### Technical Problem

As described above, bile acids, sterols, and hormones are important components as biomarker candidates. Thus, comprehensive detection of molecules of these components by MS in a biological sample is highly beneficial. Meanwhile, the polarity of molecules differs between these types of components, and there are many structural isomers with similar structures within the same type of component. Thus, conventionally, types of components to be analyzed are narrowed down in advance, and individual detection is carried out according to molecules contained therein, and a comprehensive analysis method has not yet been established.

The technique described in Non-patent Literature 1 is a technique in which analysis is carried out by using a combination of a pretreatment method such as derivatization, a plurality of separation methods (gas chromatography, liquid chromatography), and an ionization method of MS analysis (atmospheric pressure ionization method, electrospray ionization method), and has not achieved detection of components including bile acids and hormones. Further, the technique described in Non-patent Literature 2 is a technique in which MS analysis of oxysterols is carried out by using a derivatization method, the technique described in Non-patent Literature 3 is a technique in which MS analysis of steroids is carried out by using a derivatization method, and the technique described in Patent Literature 1 is a technique in which MS analysis of steroid hormones is carried out by using a derivatization method. A comprehensive analysis of components including bile acids, vitamin Ds, and thyroid hormones has not been achieved. Thus, a technique for comprehensively analyzing bile acids, sterols, and hormones has not yet been established.

An object of an aspect of the present invention is to achieve a technique for comprehensively analyzing bile acids, sterols, and hormones in a biological sample.

### Solution to Problem

In order to solve the above problem, a method for analyzing bile acids, sterols, and hormones in accordance with an aspect of the present invention includes: a step of separating a plurality of molecules selected from among bile acids, sterols, and hormones in a sample by reversed-phase liquid chromatography; a step of ionizing the molecules that have been separated; and a step of detecting, through MS analysis, the molecules that have been ionized.

A system for analyzing bile acids, sterols, and hormones in accordance with an aspect of the present invention includes: a device configured to separate a plurality of molecules selected from among bile acids, sterols, and hormones in a sample by reversed-phase liquid chromatography; and a mass spectrometry device configured to ionize the molecules that have been separated and detect, through MS analysis, the molecules that have been ionized.

### Advantageous Effects of Invention

With use of the analysis method in accordance with an aspect of the present invention, it is possible to comprehensively analyze bile acids, sterols, and hormones in a biological sample.

### Brief Description of Drawings

Fig. 1 is a block diagram illustrating a configuration of a main part of an analysis system in accordance with an embodiment of the present invention.
Fig. 2 is a view illustrating the result of MS analysis of bile acids, sterols, and hormones which were extracted from human serum in Examples.
Fig. 3 is a view illustrating the result of MS analysis of bile acids, sterols, and hormones which were extracted from human serum in Examples.
Fig. 4 is a view illustrating the result of MS analysis of bile acids, sterols, and hormones which were extracted from human crine in Examples.

### Description of Embodiments

### Method for analyzing bile acids, sterols, and hormones

An analysis method in accordance with an aspect of the present invention does not require derivatization of molecules in a sample in a pretreatment step, and includes: a step of separating molecules of bile acids, sterols, and hormones by reversed-phase liquid chromatography; a step of ionizing the molecules that have been separated; and a step of carrying out MS analysis of the ionized molecules to detect a plurality of components among bile acids, sterols, and hormones.

### <Sample>

The sample to be subjected to the present analysis method is not particularly limited, but may be a sample which may contain bile acids, sterols, and hormones. In addition, the sample may be a sample collected from a human, an animal, a plant, or a biological sample prepared from a living body. Furthermore, the biological sample can be one selected from the group consisting of a skin, a nail, an organ, a tissue, a cell, blood, urine, cerebrospinal fluid, feces, and cecal contents.

Further, the biological sample may be hair. The hair is also referred to as crine and includes head hair and body hair. Further, the hair includes not only a hair shaft portion outside a skin and a hair root portion inside the skin, but also portions, such as a root sheath or a hair mother, which are involved in the production of hair and which are present around a hair root in a hair follicle.

### <Extraction>

The sample to be analyzed may be a sample extracted from a living body. Such extraction of the sample from the living body can be realized by a known method such as solvent extraction or solid phase extraction. That is, the analysis method in accordance with an aspect of the present invention may include a step of, prior to the separation step, extracting molecules of bile acids, sterols, and hormones from a biological sample.

A solvent for use in extracting the sample is preferably a solvent having high solubility with the bile acids, sterols, and hormones which are target substances. Examples of such a solvent include methanol, heptane, ethyl acetate, butanol, chloroform, and the like.

An example of a method for comprehensively extracting molecules of bile acids, sterols, and hormones from a biological sample is presented below. First, the biological sample is suspended in the solvent, and stirred. At that time, in a case where the biological sample is a solid sample, the biological sample of about 1 mg to 10 mg is suspended in the solvent, and, in a case where the biological sample is a liquid sample, the biological sample of about 10 pL to 100 pL is suspended in the solvent. Next, after a mixed solution of the biological sample and the solvent has been stirred, centrifugation is carried out, and a supernatant is collected. The collected supernatant can be a sample to be subjected to analysis by a reversed-phase liquid chromatography mass spectrometer (LC-MS).

In a case where contaminant components are contained in an extract obtained using a solvent, these contaminant components may be removed by carrying out purification by a saponification reaction treatment or the like. For example, in a case where molecules of bile acids, sterols, and hormones are extracted from a biological sample, contaminants such as phospholipids and neutral fats can be contained. On the other hand, in a case where the biological sample is hair or the like, the amount of contaminants contained in a resultant extract is relatively small. Thus, the saponification reaction treatment does not necessarily have to be carried out.

Examples of a method of extraction from a biological sample containing a large amount of contaminants and a purification method include methods presented below. First, a biological sample is suspended in an organic solvent, and then water and potassium hydroxide are added. At this time, in a case where the biological sample is a solid sample, the biological sample of about 1 mg to 10 mg is suspended in the solvent, and, in a case where the biological sample is a liquid sample, the biological sample of about 10 pL to 100 pL is suspended in the solvent. In addition, it is preferable that the proportion of the organic solvent in the biological sample-potassium hydroxide mixed solution be 70%, and the final concentration of the potassium hydroxide be about 0.1 mol/L to 2.0 mol/L.

Next, the biological sample-potassium hydroxide mixed solution is stirred, and a saponification reaction (hydrolysis reaction) of contaminants such as phospholipids and neutral fats in the biological sample is carried out. The stirring condition is, for example, 1000 rpm to 2000 rpm, 40°C to 80°C, and 1 to 12 hours. The saponification reaction can be stopped by adding acetic acid and water.

After the saponification reaction, the molecules of bile acids, sterols, and hormones are collected by a liquid-liquid extraction method. For this liquid-liquid extraction method, butanol, heptane, and ethyl acetate can be used. For example, in a case where butanol is added to the mixed solution after the saponification reaction and stirred, the butanol to be used is 0.5 to 1.0-fold volume of butanol. Further, the stirring condition of the mixed solution is, for example, 1000 rpm to 2000 rpm.

Next, a mixture of heptane and ethyl acetate is added and stirred, and centrifugation is carried out. The stirring condition of the mixed solution is, for example, 1000 rpm to 2000 rpm. After separation into two layers has been carried out, an organic layer of an upper layer containing bile acids, sterols, and hormones is collected (upper layer 1). To the remaining lower layer, heptane and ethyl acetate are added again, and stirring and centrifugation are carried out. After separation into two layers has been carried out, an upper layer is collected (upper layer 2). A mixture of heptane and ethyl acetate to be used is, for example, 0.5 to 1.0-fold volume of a mixture of heptane and ethyl acetate. Further, the volume ratio between heptane and ethyl acetate is, for example, 3:1.

The upper layers 1 and 2 thus obtained are combined into the same tube and are then dried and solidified by nitrogen. After the drying and solidification, a resultant substance is redissolved with a mixed solution of methanol, chloroform, and water, so that a sample to be subjected to LC-MS analysis can be obtained. In the above-mentioned extraction step, an isotopically labeled standard of bile acids, sterols, and hormones may be used as an internal standard substance, if necessary. Examples of the internal standard substance include 7α,25-dihydroxycholesterol_d6, 25-hydroxycholesterol_d6, testosterone_¹³C₃, and the like.

### <Bile acids, sterols, and hormones>

The bile acids, sterols, and hormones detected in the present analysis method are components mainly containing cholesterol as a precursor. The bile acids, sterols, and hormones are components selected from the group consisting of bile acids, cholesterol analogs, oxysterols, vitamin Ds, hormones, and thyroid hormones. The cholesterol analogs include cholesterols, cholesterol precursors, and phytosterols. The bile acids, sterols, and hormones may be bile acids, sterols, and hormones biosynthesized in vivo, or may be exogenous bile acids, sterols, and hormones from a plant or the like.

Examples of the cholesterols include cholesterol, latosterol, desmosterol, zymosterol, zymostanol, lanosterol, dehydrolanosterol, coprostanol, 7-dehydrocholesterol, FF-MAS, dihydro FF-MAS, T-MAS, dihydro T-MAS, squalene, cholestenone, cholestanol, coprostanol, and the like. Examples of the phytosterols include campesterol, campestanol, sitosterol, sitostanol, brassicasterol, stigmasterol, and the like. Examples of the oxysterols include 7-ketocholesterol, 7α-hydroxycholesterol, 7β-hydroxycholesterol, 5α,6α-epoxycholestanol, 5β,6β-epoxycholestanol, 24 (S),2 5-epoxycholesterol, 25-hydroxycholesterol, 27-hydroxycholesterol, 19-hydroxycholesterol, 20α-hydroxycholesterol, 22(S)-hydroxycholesterol, 22 (R) -hydroxycholesterol, 4β-hydroxycholesterol, 24(S)-hydroxycholesterol, 24 (R)-hydroxycholesterol, 5α,6β-dihydroxycholestanol, 7α,25-dihydroxycholesterol, 7α,27-dihydroxycholesterol, 7α,24(S)-dihydroxycholesterol, 7β,25-dihydroxycholesterol, 7β,27-dihydroxycholesterol, 7α-hydroxy-3-oxocholesterol acid, 7α-hydroxycholestenone, and the like. Examples of the vitamin Ds include ergosterol, vitamin D3, 25-hydroxyvitamin D3, 1α,25-dihydroxyvitamin D3, 24(S),25-dihydroxyvitamin D3, vitamin D2, 25-hydroxyvitamin D2, and the like. Examples of the hormones include dehydroepiandrosterone, dehydroepiandrosterone sulfate, testosterone, 5α-dehydrotestosterone, estrone, 17β-estradiol, estriol, progesterone, 16,17-epoxyprogesterone, 17-hydroxyprogesterone, cortisone, cortisol, corticosterone, aldesterone, 11-deoxycorticosterone, 11-deoxycortisol, androsterone, androstenedione, pregnenolone, 17-hydroxypregnenolone, and the like. Examples of the bile acids include cholic acid, chenodeoxycholic acid, ursocholic acid, hyocholic acid, muricolic acid, glycocholic acid, glycochenodeoxycholic acid, taurocholic acid, taurochenodeoxycholic acid, deoxycholic acid, hyodeoxycholic acid, ursodeoxycholic acid, glycodeoxycholic acid, glycoursodeoxycholic acid, taurodeoxycholic acid, tauroursodeoxycholic acid, lithocholic acid, glycolithocholic acid, taurolithocholic acid, 3-ketolithocholic acid, 7-ketolithocholic acid, and the like. Examples of the thyroid hormones include triiodothyronine (T3), reverse T3, thyroxine, and the like. The bile acids, sterols, and hormones detected in the analysis method can also include isomers of the molecules described above, sulfate conjugates, and glucuronide conjugates, and the like.

The present analysis method enables molecules belonging to the above-described bile acids, sterols, and hormones to be comprehensively detected. A plurality of components detected in this analysis method may be, for example, a combination of cholesterol and latosterol, which are among cholesterols, and a combination of cholesterol and campesterol, which is among phytosterols. Further, the detected components may be a combination of isomers, such as 7α-hydroxyoxysterol and 7β-hydroxyoxysterol.

The number of bile acids, sterols, and hormones detected in the analysis method is not particularly limited and only needs to be not less than 2, but may be not less than 5, not less than 10, not less than 15, not less than 20, not less than 30, not less than 40, not less than 50, and not less than 60. Comprehensively analyzing many molecules at a time enables achievement of high-throughput analysis.

### (Separation step)

In the separation step, molecules of bile acids, sterols, and hormones in a sample are separated by reversed-phase liquid chromatography. In this separation step, the sample is subjected to reversed-phase liquid chromatography to separate bile acids, sterols, and hormones on a molecule-by-molecule basis. The sample to be subjected to the separation step is preferably a sample adjusted to a state suitable for reversed-phase liquid chromatography, and is, for example, a sample obtained by extraction with a solvent. In addition, in the separation step, the sample is separated by reversed-phase liquid chromatography under acidic conditions.

The separation step eliminates the need to divide the sample into bile acids, sterols, and hormones and the need to change any separation conditions. Carrying out the separation step once enables bile acids, sterols, and hormones contained in the same sample to be separated on a component-by-component basis.

The particle diameter of a filler in a column for reversed-phase liquid chromatography is more preferably not more than 3 pm, and most preferably not more than 2 pm.

Further, the filler in the column for reversed-phase liquid chromatography is preferably a filler for ultra-high performance liquid chromatography.

The solvent used in the reversed-phase liquid chromatography under acidic conditions may be a mixed solvent of water and an organic solvent. As the organic solvent, for example, methanol, acetonitrile, 2-propanol, or the like can be suitably used. In addition, in the reversed-phase liquid chromatography, the above-described mixed solvent is made acidic with use of a volatile acid. The volatile acid is, for example, acetic acid, formic acid, or the like. The proportion of the volatile acid to the organic solvent may be, for example, not less than 1%.

The flow rate of a mobile phase in the reversed-phase liquid chromatography is around 0.4 mL/min, and the temperature of a column oven is preferably not lower than 40°C and not higher than 50°C.

In the separation step, the separation of each molecule of bile acids, sterols, and hormones in the sample can be carried out in a gradient solvent using two types of mobile phases. Examples of a composition of such mobile phases include a mixture of water and methanol (containing 1% acetic acid), a mixture of methanol and acetonitrile (containing 1% acetic acid), and the like.

### (Ionization step)

In the ionization step, the molecules separated by the reversed-phase liquid chromatography are ionized by MS. For example, the molecules are ionized by an electrospray ionization method (ESI method).

An ion source for the ionization in the ionization step is, for example, an ESI probe. In this ionization step, it is preferable that the temperatures of the ion source, an orifice, and an ion introduction part be set to 400°C to 500°C, 250°C to 400°C, and 200°C to 300°C, respectively.

In addition, in the ionization step, an ionization promoter for promoting the ionization of bile acids, sterols, and hormones is preferably used. Examples of such an ionization promoter include volatile acids such as acetic acid and formic acid. Further, examples of a carrier gas used in the ionization step include nitrogen gas.

### (Detection step)

In the detection step, the ionized molecules are subjected to MS analysis to detect a plurality of components among bile acids, sterols, and hormones contained in the sample. In this detection step, the ionized molecules are analyzed with use of a known mass spectrometry device such as a triple quadrupole MS.

In the detection step, it is not necessary to change the mass spectrometry device for each of the bile acids, the sterols, and the hormones, and carrying out MS analysis once enables a plurality of components among bile acids, sterols, and hormones contained in the sample to be comprehensively detected.

In the detection step, MS and MS/MS analysis of the ionized bile acids, sterols, and hormones are carried out, and a plurality of components among bile acids, sterols, and hormones are each identified according to an obtained mass-to-charge ratio (m/z ratio).

### (Operation step)

The method for analyzing the bile acids, the sterols, and the hormones may further include an operation step of quantifying the bile acids, the sterols, and the hormone molecules in the sample on the basis of a detection result in the detection step. In the operation step, the quantification may be carried out by generating the MS and MS/MS spectra of each component in the sample on the basis of the detection result in the detection step, and measuring a peak area.

According to the above-described method for analyzing bile acids, sterols, and hormones, it is possible to analyze a plurality of components among bile acids, sterols, and hormones contained in the sample by a single reversed-phase liquid chromatography, a single ionization, and a single detection processing step. That is, the present analysis method enables achievement of comprehensive analysis of bile acids, sterols, and hormones simultaneous analysis of which was conventionally difficult to carry out. Therefore, the present analysis method eliminates the need to individually detect bile acids, sterols, and hormones, and enables achievement of high-throughput analysis.

Bile acids, sterols, and hormones, which are important lipid components, are correlated with various diseases as well as health maintenance, and are very useful as biomarker candidates. The present analysis method enables comprehensive analysis of a plurality of components among bile acids, sterols, and hormones, and is thus effective for searching and discovering a molecule that serves as a biomarker.

### System for analyzing bile acids, sterols, and hormones

An analysis system in accordance with an aspect of the present invention includes: a device configured to separate molecules of bile acids, sterols, and hormones in a sample by reversed-phase liquid chromatography; and a mass spectrometry device configured to ionize the molecules that have been separated and subject, to MS analysis, the molecules that have been ionized to detect these plurality of components. That is, the above-described method for analyzing bile acids, sterols, and hormones is an aspect of an analysis method that is carried out in the system for analyzing bile acids, sterols, and hormones in accordance with the present invention.

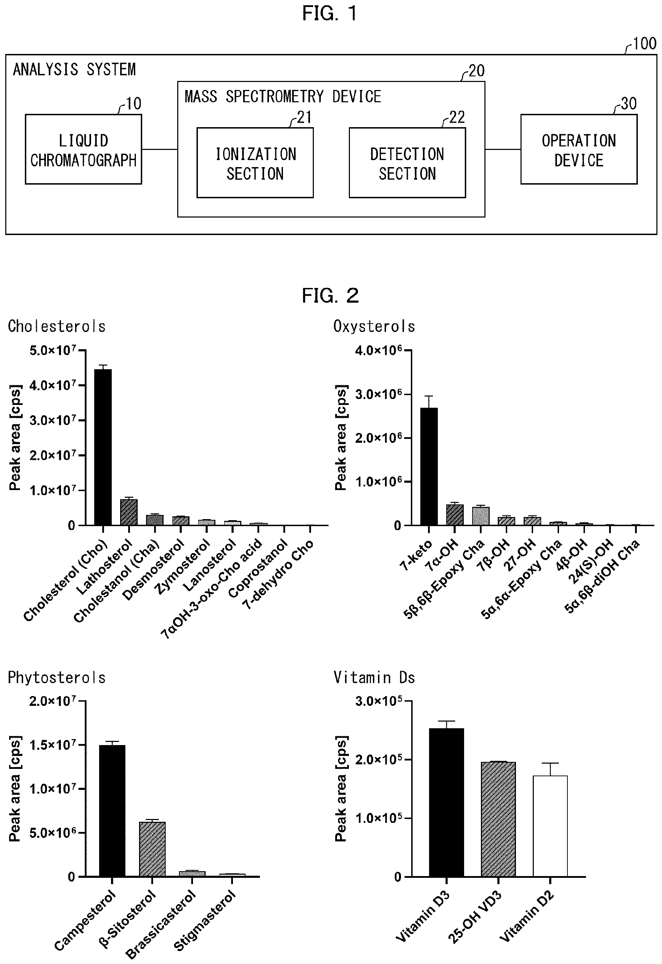

Fig. 1 is a block diagram illustrating a configuration of a main part of an analysis system in accordance with an embodiment of the present invention. As illustrated in Fig. 1, an analysis system 100 includes a liquid chromatograph (separation device) 10, a mass spectrometry device 20, and an operation device 30. Note that, in the analysis system 100, the liquid chromatograph 10 and the mass spectrometry device 20 may be respectively independent devices or may be an integrated device that includes the function of the liquid chromatograph 10 and the function of the mass spectrometry device 20. As the integrated device that includes the function of the liquid chromatograph 10 and the function of the mass spectrometry device 20, a known reversed-phase liquid chromatography mass spectrometer (LC-MS) can be used.

### (Liquid chromatograph)

The liquid chromatograph 10 is a device configured to separate a sample by reversed-phase liquid chromatography. The liquid chromatograph 10 may be a liquid chromatograph device known in the art, and a commercially available device can be suitably used. The sample to be separated by the liquid chromatograph 10 may be a sample adjusted to a state suitable for separation in the liquid chromatograph 10, and is, for example, a solvent extraction liquid obtained by extracting a sample with a solvent.

With the liquid chromatograph 10, it is possible to separate molecules of bile acids, sterols, and hormones, which are target components in a sample, on a component-by-component basis. The components separated by the liquid chromatograph 10 are introduced into the mass spectrometry device 20.

### (Mass spectrometry device)

The mass spectrometry device 20 is a device configured to carry out MS analysis of bile acids, sterols, and hormones in a sample. The mass spectrometry device 20 includes an ionization section 21 configured to ionize molecules that have been separated by the liquid chromatograph 10, and a detection section 22 configured to carry out MS analysis of the molecules that have been ionized to detect a plurality of components among bile acids, sterols, and hormones contained in a sample. The mass spectrometry device 20 may be a mass spectrometry device known in the art, and a commercially available device can be suitably used.

The sample introduced into the mass spectrometry device 20 is first introduced into the ionization section 21, and molecules of bile acids, sterols, and hormones in the sample are ionized. The detection section 22 separates the molecules that have been ionized in the ionization section 21, according to the mass-to-charge ratio (m/z ratio), and detects the molecules. A detection result obtained by the mass spectrometry device 20 is outputted to the operation device 30.

### (Operation device)

The operation device 30 generates MS and MS/MS spectra of the bile acids, sterols, and hormones in the sample on the basis of the detection result outputted from the mass spectrometry device 20, and measures peak areas, thereby quantifying the molecules of the bile acids, sterols, and hormones in the sample.

The operation device 30 may be, for example, a computer into which software for performing an operation related to MS analysis is incorporated and which includes, for example, a memory for storing an operation result and a display for displaying the operation result.

### <Software Implementation Example>

The operation performed by the operation device 30 can be realized by a logic circuit (hardware) provided in an integrated circuit (IC chip) or the like or can be alternatively realized by software as executed by a central processing unit (CPU).

In the latter case, the operation device 30 includes a CPU that executes instructions of a program that is software realizing the foregoing functions; a read only memory (ROM) or a storage device (each referred to as "storage medium") in which the program and various kinds of data are stored so as to be readable by a computer (or a CPU); and a random access memory (RAM) in which the program is loaded. An object of the present invention can be achieved by a computer (or a CPU) reading and executing the program stored in the storage medium. Examples of the storage medium encompass "a non-transitory tangible medium" such as a tape, a disk, a card, a semiconductor memory, and a programmable logic circuit. The program can be made available to the computer via any transmission medium (such as a communication network or a broadcast wave) which allows the program to be transmitted. Note that the present invention can also be achieved in the form of a computer data signal in which the program is embodied via electronic transmission and which is embedded in a carrier wave.

According to the analysis system 100 for analyzing bile acids, sterols, and hormones, it is possible to analyze a plurality of components among bile acids, sterols, and hormones contained in the sample by a single reversed-phase liquid chromatography, a single ionization, and a single detection processing step.

The present invention is not limited to the embodiments, but can be altered by a skilled person in the art within the scope of the claims. The present invention also encompasses, in its technical scope, any embodiment derived by combining technical means disclosed in differing embodiments. Examples

Bile acids, sterols, and hormones in crine and serum were analyzed simultaneously as presented below.

### Extraction

1 mg of crine collected from healthy male and female human samples was suspended in 200 pL of methanol, and then 100 pL of chloroform and 20 pL of water were added and stirred. After the mixed solution had been stirred, centrifugation was carried out, and a supernatant was collected in a measurement vial for an MS analyzer. The collected supernatant was subjected to analysis by reversed-phase LC-MS.

After suspending 20 pL of commercially available human standard serum (manufactured by Kohjin Bio Co., Ltd.; Lot Number: BJ10290A) in 90 pL of methanol, 10 pL of water and 8 pL of 8 mol/L potassium hydroxide were added. The mixed solution was stirred, and a saponification reaction of contaminants was carried out. The saponification reaction was stopped by adding acetic acid and water.

Thereafter, the sample was collected by a liquid-liquid extraction method using butanol, heptane, and ethyl acetate. First, butanol was added, followed by stirring. Then, a mixture of heptane and ethyl acetate was added, followed by stirring, and centrifugation was carried out. After separation into two layers had been carried out, an organic layer of an upper layer was collected (upper layer 1). To the remaining lower layer, a mixture of heptane and ethyl acetate was added again, and stirring and centrifugation were carried out. After separation into two layers had been carried out, an upper layer was collected (upper layer 2). The upper layers 1 and 2 thus obtained were combined into the same tube and were then dried and solidified by nitrogen. After the drying and solidification, a resultant substance was redissolved with a mixed solution of methanol, chloroform, and water, and a resultant sample was subjected to LC-MS analysis.

### Separation

By an ultra-high performance liquid chromatograph NEXERA X2 (manufactured by Shimadzu Corporation), separation of the extracted sample by reversed-phase liquid chromatography was carried out as follows. As a filler in a column for reversed-phase liquid chromatography, a reversed-phase filler for ultra-high performance liquid chromatography was used. Gradient elution was carried out with use of two types of mobile phases as mobile phases used in reversed-phase liquid chromatography under acidic conditions. As the mobile phases, a mixed solvent of water and methanol and a mixed solvent of methanol and acetonitrile were used. Acetic acid was added to each of the mixed solvents in a proportion of 1% relative to methanol. The flow rate of the mobile phase in reversed-phase liquid chromatography was 0.4 mL/min, and the temperature of a column oven was 40°C.

### Ionization

Ionization of the separated molecules was carried out by an electrospray ionization method (ESI method) in a triple quadrupole LCMS-8060 system (manufactured by Shimadzu Corporation). The temperatures of an ion source, an orifice, and an ion introduction part were set to 450°C to 500°C, 350°C to 400°C, and 250°C to 300°C, respectively. Acetic acid was used as an ionization promoter, and nitrogen gas was used as a carrier gas.

### Detection

The ionized molecules were subjected to MS-analysis in the LCMS-8060 system described above to identify and quantify the components which are bile acids, sterols, and hormones.

### Results

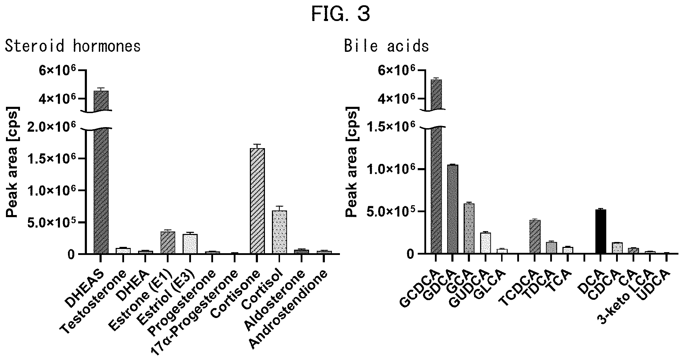

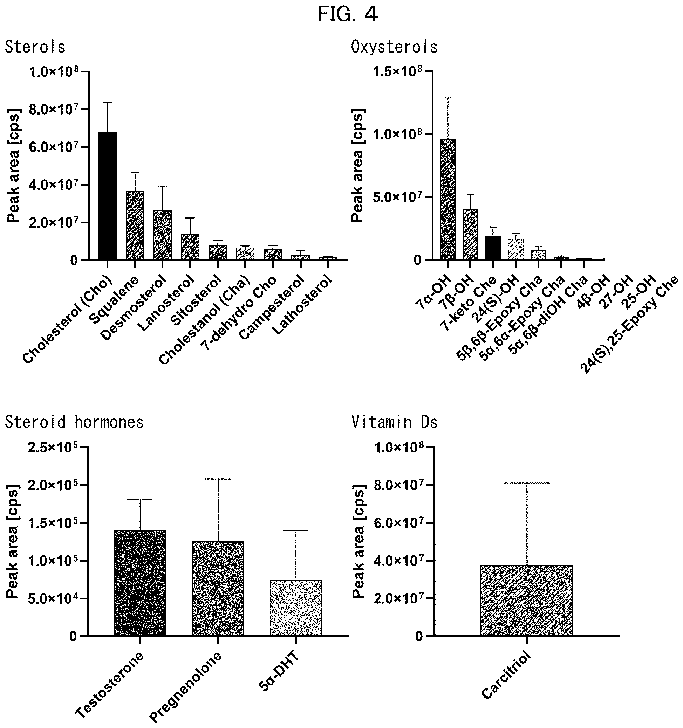

As illustrated in Figs. 2 to 3 and Table 1, 49 types of bile acids, sterols, and hormones in the sample extracted from human serum could be comprehensively MS-analyzed. As illustrated in Fig. 4 and Table 2, 24 types of sterols and hormones in the sample extracted from human crine could be comprehensively MS-analyzed.

**[Table 1]**

| **Group** | **Molecule** | **Mean** | **SD** |
|---|---|---|---|
| ***Oxysterols*** | 24(S)- OH | 2.0.E+04 | 4.2.E+03 |
| | 27-OH | 1.9.E+05 | 2.9.E+04 |
| | 7β-ON | 1.9.E+05 | 2.9.E+04 |
| | 7α-OH | 4.9.E+05 | 4.6.E+04 |
| | 4β-OH | 5.1.E+04 | 1.4.E+04 |
| | 5β,6β-Epoxy Cha | 4.3.E+05 | 3.4.E+04 |
| | 5α,6α-Epoxy Cha | 8.2.E+04 | 3.4.E+03 |
| | 5α,6β-diOH Cha | 2.2.E+04 | 2.9.E+03 |
| | 7-keto Ch | 2.7.E+06 | 2.7.E+05 |
| ***Sterols*** | Cholesterol (Cho) | 4.5.E+07 | 1.3.E+06 |
| | Desmosterol | 2.6.E+06 | 8.7.E+04 |
| | Zymosterol | 1.7.E+06 | 3.2.E+04 |
| | Gathosterol | 7.6.E+06 | 5.2.E+05 |
| | 7-dehydro Cho | 1.7.E+05 | 9.1.E+03 |
| | Cholestanol (Cha) | 3.1.E+06 | 3.1.E+05 |
| | 7αOH-3-oxo-Cho acid | 6.0.E+05 | 7.0.E+04 |
| | Co prostanol | 1.3.E+05 | 2.6.E+04 |
| | Lanosterol | 1.3.E+06 | 1.2.E+05 |
| ***Phytosterols*** | Campestrol | 1.5.E+07 | 4.4.E+05 |
| | β-Sitostemol | 6.3.E+06 | 2.5.E+05 |
| | Brassicasterol | 6.6.E+05 | 6.5.E+04 |
| | Stigmasterol . | 3.5.E+05 | 2.4.E+04 |
| ***Vitamin Ds*** | Vitamin D3 | 2.5.E+05 | 1.2.E+04 |
| | 25-OHVD3 | 2.0.E+05 | 6.8.E+02 |
| | Vitamin D2 | 1.7.Et05 | 2.1.E+04 |
| ***Steroid hormones*** | DHEAS | 4.6.E+06 | 2.0.E+05 |
| | Cortisone | 1.7.E+06 | 5.8.E+04 |
| | Cortisol | 6 9.E+05 | 6.1.E+04 |
| | Estrone (E1) | 3.6.E+05 | 2.7.E+04 |
| | Estriol (E3) | 3.2 E+05 | 2.8.E+04 |
| | Testosterone | 9.9.E+04 | 1.1.E+04 |
| | Aldosterone | 7.3.E+04 | 1.2.E+04 |
| | DHEA | 5.3.E+04 | 1.0.E+04 |
| | Androstendione | 5.4.E+04 | 1.0.E+04 |
| | Progesterone | 4.1.E+04 | 1.2.E+04 |
| | 17αOH-Progesterone | 1.9.E+04 | 5.4.E+03 |
| ***Bite acids*** | GCDCA | 5.3.E+06 | 1.3.E+05 |
| | GOCA | 1.0.E+06 | 8.3.E+03 |
| | GCA | 6.0.E+05 | 1.6 E+04 |
| | GUDCA | 2.5.E+05 | 1.0.E+04 |
| | GLCA | 6.0.E+04 | 4.9.E+03 |
| | TCDCA | 4.0.E+05 | 1.2.E+04 |
| | TDCA | 1.4.E+05 | 1.0.E+04 |
| | TCA | 8.3.E+04 | 6.9, E+03 |
| | DCA | 5.2.E+05 | 1.1.E+04 |
| | CA | 6.9.E+04 | 6.1.E+03 |
| | CDC4 | 1.4.E+05 | 1.5.E+03 |
| | UDCA | 1.1.E+04 | 3.3.E+03 |
| | 3-keto LCA | 3.0.E+04 | 9.2.E+02 |

**[Table 2]**

| Group | Molecule | Male Mean | SD | Female Mean | SD |
|---|---|---|---|---|---|
| Sterols | Cholesterol (Ch) | 6.79.E+07 | 1.58.E+07 | 5.68.E+07 | 1.05.E+07 |
| | Lathosterol | 1.61.E+06 | 5.89.E+05 | 1.21.E+06 | 2.31.E+05 |
| | Lanosterol | 1.41.E+07 | 8.31.E+06 | 1.17.E+07 | 6.43.E+06 |
| | 7-dehydro cholesterol | 6.05.E+06 | 1.84.E+06 | 5.19.E+06 | 1.16.E+06 |
| | Desmosterol/Zymosterol | 2.64.E+07 | 1.30.E+07 | 1.80.E+07 | 1.43.E+07 |
| | Cholestanol (Cha) | 6.78.E+06 | 8.39.E+05 | 4.25.E+06 | 1.16.E+06 |
| Oxysterols | 25-OH Ch | 1.33.E+06 | 6.41.E+05 | 1.04.E+06 | 5.60.E+05 |
| | 24(S)-OH Ch | 1.69.E+07 | 4.17.E+06 | 1.44.E+07 | 4.99.E+06 |
| | 27-OH Ch | 3.11.E+05 | 1.33.E+05 | 2.30.E+05 | 7.41.E+04 |
| | 70-0 H Ch | 4.03.Et07 | 1.19.E+07 | 5.02.E+07 | 3.33.E+07 |
| | 7α-OH Ch | 9.61.E+07 | 3.26.E+07 | 9.07E+07 | 4.18E+07 |
| | 4β-OH Ch | 6.10.E+05 | 3.40.E+05 | 7.50.E+05 | 3.29.E+05 |
| | 5β,6β-epoxy Cha | 7.73.Et06 | 2.83.E+06 | 9.41.E+06 | 4.85.E+06 |
| | 5α, 6α- epoxy Cha | 2.30.E+06 | 8.69.E+05 | 2.15.E+06 | 1.08.E+06 |
| | 24(S),25-epoxy Ch | 6.89.E+05 | 4.21.E+05 | 5.91.E+05 | 5.75.E+05 |
| | 7-kato Ch | 1.94.E+07 | 7.00.E+06 | 3.66.E+07 | 3.25.E+07 |
| | 5α,6β-diOH Ch | 1.17.E+06 | 1.70.E+05 | 1.55.E+06 | 1.47.E+06 |
| Phytosterols | Cam pesterol | 2.80.E+06 | 2.19.E+06 | 1.41.E+06 | 4.49.E+05 |
| | Sitosterol | 8.18.E+06 | 2.43E+06 | 8.20.E+06 | 4.06.E+06 |
| Steroid hormones | Teststoerone | 1.41.E+05 | 3.93.E+04 | 3.43.E+05 | 4.83.E+05 |
| | 5α-DHT | 7.40.E+04 | 6.58.E+04 | 7.53.E+04 | 3.92.E+04 |
| | Pregnenolone | 1.25.E+05 | 8.28.E+04 | 1.85.E+05 | 1.28.E+05 |
| *Vitamin Ds* | Calcitriol | 3.76.E+07 | 4.37.E+07 | 7.56.E+07 | 1.06.E+08 |
| Other | Squalane | 3.69.E+07 | 9.63.E+06 | 3.45.E+07 | 4.93.6+07 |

### Industrial Applicability

The present invention is applicable to a medical field or the like for predicting or diagnosing health conditions or diseases.

### Reference Signs List

- 10: liquid chromatograph (analysis device)
- 20: mass spectrometry device
- 21: ionization section
- 22: detection section
- 30: operation device
- 100: analysis system

## Claims

1. A method for analyzing bile acids, sterols, and hormones, said method comprising:
a step of separating a plurality of molecules selected from among bile acids, sterols, and hormones in a sample by reversed-phase liquid chromatography;
a step of ionizing the molecules that have been separated; and
a step of detecting, through MS analysis, the molecules that have been ionized.

2. The method according to claim 1, wherein, in the separating step, the molecules of the bile acids, the sterols and the hormones in the sample are separated by reversed-phase liquid chromatography under acidic conditions.

3. The method according to claim 1 or 2, wherein, in the ionizing step, the molecules are ionized by an ESI method.

4. The method according to any one of claims 1 to 3, wherein the sample in the separating step is an extract from a biological sample collected from a living body.

5. The method according to any one of claims 1 to 3, further comprising a step of, prior to the separating step, performing solvent extraction or solid phase extraction to extract the sample from a biological sample collected from a living body.

6. The method according to claim 4 or 5, wherein the biological sample is selected from the group consisting of a skin, a nail, an organ, a tissue, a cell, blood, urine, cerebrospinal fluid, feces, and cecal contents.

7. The method according to claim 4 or 5, wherein the biological sample is hair.

8. The method according to any one of claims 1 to 7, wherein the bile acids, the sterols, and the hormones are components selected from the group consisting of bile acids, cholesterol analogs, oxysterols, vitamin Ds, hormones, and thyroid hormones.

9. A system for analyzing bile acids, sterols, and hormones, said system comprising:
a device configured to separate a plurality of molecules selected from among bile acids, sterols, and hormones in a sample by reversed-phase liquid chromatography; and
a mass spectrometry device configured to ionize the molecules that have been separated and detect, through MS analysis, the molecules that have been ionized.
